# EUROPEAN PATENT APPLICATION

(11) **EP 2 808 012 A1**
(43) Date of publication of application: **03.12.2014**
(21) Application number: 13002791.5
(22) Date of filing: 29.05.2013
(51) Int. Cl.: A61K 9/20, A61K 31/165

(54) **Method for producing dosage form comprising odanacatib**

(71) Applicant: ratiopharm GmbH, 89079 Ulm (DE)
(72) Inventor: Rimkus, Katrin, 82049 Pullach (DE); Holfinger, Konstantin, 81379 München (DE)
(74) Representative: Ter Meer Steinmeister & Partner

(57) **Abstract**

The present invention relates to a method for producing a dosage form, preferably a dosage form for immediate release, containing odanacatib wherein the method comprises the granulation with a specific granulation fluid. The invention further relates to a dosage form obtained according to said method.

## Description

### Background of the Invention

The present invention relates to a method for producing a dosage form, preferably a dosage form for immediate release, containing odanacatib, wherein the method comprises granulation, preferably wet granulation with a specific granulation fluid. The invention further relates to a dosage form obtained according to said method.

"Odanacatib" is reported to be the INN name of (2*S*)-*N*-(1-cyanocyclopropyl)-4-fluor-4-methyl-2-[[(1*S*)-2,2,2-trifluor-1-[4-(4-methylsulfonylphenyl)phenyl]ethyl]amino]pentanamid and is characterized by the following chemical formula (I):

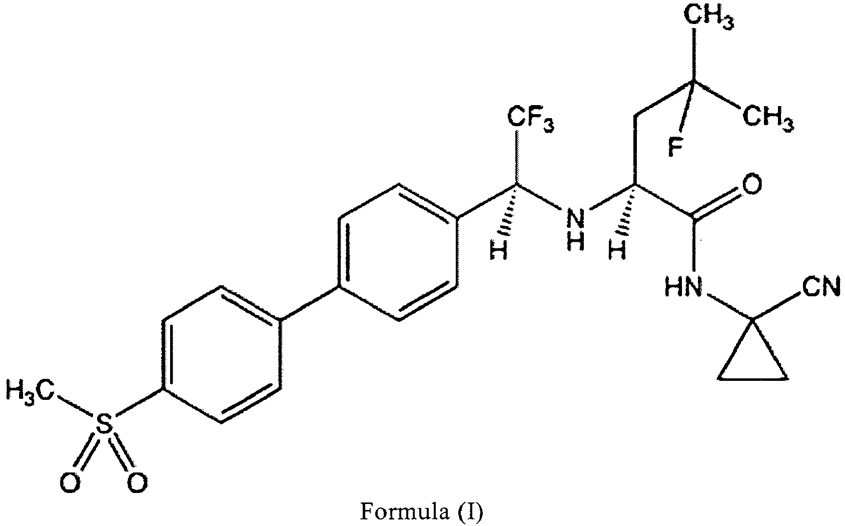

Odanacatib is considered to selectively inhibit the enzyme cathepsin, in particular cathepsin K. Cathepsin K, a lysosomal cysteine protease being expressed by osteoclasts during the process of bone resorption, acts as the major collagenase responsible for the degradation of the organic bone matrix during the bone remodelling process. Odanacatib is reported to increase the bone mineral density and to reduce bone turnover markers in particular in post-menopausal women with low bone mineral density. For this purpose, odanacatib is suggested to be used in the treatment of osteoporosis and bone metastasis, in particular in the treatment of post-menopausal osteoporosis.

The synthesis of odanacatib has previously been described, for example in WO 03/075836 and WO 2006/017455. However, these documents allegedly describe processes for the generation of the crystalline form of odanacatib, which is described as being poorly soluble. For example WO 2008/106059 allegedly describes formulations of odanacatib having an undesirable slow release profile.

To improve the slow release the proprietor of WO 2008/106059 describes its further development in application WO 2009/140105, relating to a tablet containing odanacatib showing an improved dissolution , wherein the tablets are produced by spray-drying for converting the active agent into amorphous form and subsequent compaction. This development is set forth in the recent WO 2013/025449 relating to methods for increasing the solubility of active substances having poor water solubility. For this purpose the active substance is, for example, milled in the presence of an inorganic matrix and a secondary polymer to convert the crystalline active substance to substantially amorphous and stable form, i.e. the crystallinity is less than 5%. However, these processes tend to suffer from the disadvantages known in the handling of amorphous active pharmaceutical agents, such as reduced flowability. Further, as known from the example from the "Handbook of Fruit and Vegetable Flavors" by Y. H. Hui, Feng Chen Leo M. L. Nollet, TABLE 26.4, spray-drying bears the risk of creating high drying costs, of damaging certain products, and of being prone to humidity.

Additionally, the teaching of WO 2009/140105 only results in tablets having a low drug load of about 6.5 wt%. In order to generate for example a tablet having a content of 50 mg of odanacatib, such tablet as described in WO2009/140105 would have a weight of about 750 mg. Thus, the tablet would be undesirable large in size and therefore would be difficult to swallow. In particular, older people like post-menopausal women might show a poor patient compliance due to the size of the tablet.

Thus, the known compositions were not suitable for production of dosage forms having a higher drug load or an appropriately high dissolution profile. Further, it turned out that the API (odanacatib) in its spray-dried form is difficult to handle and difficult to process. In particular, the flowability problems of the compositions tend to get worse the higher the content of the active pharmaceutical agent is.

Hence, it was an object of the present invention to overcome the above problems.

An object of the present invention is to provide a dosage form, preferably a tablet, containing odanacatib with dissolution properties similar or equal to the ones of the tablets as described in WO 2009/140105, but having a higher drug-load, therefore smaller size than the tablets according to WO 2009/140105.

Additionally, the dosage form should have a suitable size which enables administering an effective amount (e.g. 25, 50, or 100 mg per dosage form) to patients, preferably mammals, e.g. post-menopausal women, potentially leading to a superior patient compliance.

Further, a tablet should be provided having superior high content uniformity, superior friability, superior hardness and/or superior stability with regard to the state of art.

Another object of the invention is the avoidance of organic solvents in the production process. Both alleged patent applications, WO 2013/025449 and WO 2009/140105, use organic solvents in the described formulation processes. However, it has been extensively described that high amounts of organic solvents are harmful to the environment and the employees. Thus, processes using such solvents require higher investments in protective measurements and should therefore be avoided.

Another object of the invention was to provide a more cost and time effective process for formulation as those described in WO 2013/025449 and WO 2009/140105.

### Summary of the Invention

Several technologies are generally known to enhance the solubility of poorly soluble compounds. One of these technologies is amorphization. However, especially amorphization have been shown to have certain drawbacks, e.g. low flowability. In addition, the effect of these technologies is not ubiquitously enhancing it rather depends strongly on the API and the formulation.

The processes described in WO 2013/025449 and WO 2009/140105 lead to tablets having a high weight and low drug load. Furthermore, these processes are costly in respect of time and money and in addition rely on the usage of solvents potentially harmful to the environment.

The objectives described above are achieved by the present invention which pertains to a specific process that inter alia comprises the phase of granulating a mixture of odanacatib and at least one pharmaceutical excipient with a granulation liquid, the latter being preferably essentially free of organic solvent and optionally comprises a surfactant and optionally at least one further pharmaceutical excipient, preferably a binder.

Furthermore, the above mentioned drawbacks can be overcome by a dosage form, preferably a tablet, prepared according to the method of the present invention.

Thus, the subject of the present invention is a method for producing a dosage form containing odanacatib comprising the phases of
- phase I): providing odanacatib either alone or in addition with at least one pharmaceutical excipient; and
- phase II): granulating the mixture of phase I) with a granulation liquid, preferably an aqueous granulation liquid, comprising a surfactant and optionally at least one further pharmaceutical excipient, preferably a binder; and
- phase III): blending the granulates of phase II) with further pharmaceutical excipient(s); and
- finally: processing the blend of phase III) into a dosage form.

It was found that according to the method of the present invention a dosage form can be prepared which is easy to prepare by a process less harmful for the environment, which provides a dissolution profile at least as good as the formulation described in WO 2009/140105 and which allows effective treatment including excellent patient compliance. Further, the present method results in dosage forms having superior properties such as superior release properties, content uniformity and stability.

The subject-matter of the invention also relates to a dosage form prepared according to the method of the present invention.

### Detailed Description of the Invention

The present invention relates to a method for producing a dosage form containing odanacatib comprising the phases of
- phase I): providing odanacatib either alone or in addition with at least one pharmaceutical excipient; and
- phase II): granulating the mixture of phase I) with a granulation liquid comprising a surfactant and optionally at least one further pharmaceutical excipient, preferably a binder; and
- phase III): optionally blending the granulates of phase II) with further pharmaceutical excipient(s); and
- finally: processing the blend of phase III) into a dosage form.

In the context of this invention, the term "odanacatib" usually refers to (2*S*)-*N*-(1-cyanocyclopropyl)-4-fluor-4-methyl-2-[[(1*S*)-2,2,2-trifluor-1-[4-(4-methylsulfonyl-phenyl)phenyl]ethyl]amino]pentanamid in accordance with Formula (I) above. In addition, the term "odanacatib" as used in the present application can refer to odanacatib in the form of the free base as well as to its pharmaceutically acceptable salts, hydrates, solvates, polymorphs and mixtures thereof. The pharmaceutically acceptable salts e.g. can be obtained by the reaction of odanacatib with an acid.

In a particularly preferred embodiment of the present invention odanacatib is used in the form of the free base. Unless otherwise mentioned within the present application the amounts or weight-% of odanacatib are based on the amount of odanacatib in form of the free base (i.e. if odanacatib is present in form of a salt, the amount of the salt has to be added accordingly).

In a preferred embodiment the dosage form of the present invention comprises odanacatib in an amount of 10 to 30 wt%, more preferably 12 to 25 wt%, even more preferably 12 to 20 wt%, in particular 12 to 18 wt%, based on the total weight of the dosage form.

In a preferred embodiment of the invention odanacatib is used as the sole pharmaceutically active agent.

In another preferred embodiment odanacatib can be used in combination with further pharmaceutically active agent(s).

In phase I) of the method of the invention odanacatib and at least one pharmaceutical excipient and optionally one or more additional APIs are provided.

Generally, the term "excipient" refers to any pharmaceutical acceptable excipient, preferably to those which are described in the European Pharmacopoeia and/or in the US Pharmacopoeia (USP).

Examples of excipients are fillers, disintegrants, binders, surfactants, lubricants and glidants.

Fillers can be used to increase the bulk volume and weight of a low-dose drug to a limit at which a pharmaceutical dosage can be formed. Fillers may fulfil several requirements, such as being chemically inert, non-hygroscopic, biocompatible, easily processable and possessing good biopharmaceutical properties. Examples of fillers are microcrystalline cellulose, dextrose, lactose, sucrose, glucose, mannitol, calcium carbonate, cellulose and others. Fillers can preferably be present in amounts of 50 to 85 wt%, preferably of 55 to 80 wt%, in particular of 60 to 77 wt% based on the total weight of the dosage form.

Disintegrants are reported to be compounds which can enhance the ability of the intermediate to break into smaller fragments when in contact with a liquid preferably water. Preferred disintegrants are sodium carboxymethyl starch (croscarmellose sodium) cross-linked polyvinylpyrrolidone, sodium carboxymethyl glycolate, swelling polysaccharide, for example soy polysaccharide, carrageenan, agar, pectin, starch and derivatives thereof, protein, for example formaldehyde-casein, sodium bicarbonate or mixtures thereof. Disintegrants can preferably be present in amounts of 2 to 35 wt%, preferably of 3 to 30 wt%, in particular of 3 to 25 wt% based on the total weight of the dosage form.

Binders or adhesives are reported to be substances that ensure that granulates or tablets can be formed with the required mechanical strength. Binders can be, for example, starch, hydroxy propyl cellulose, sucrose, gelatine, polyvinylpyrrolidone, cellulose derivatives, such as hydroxypropyl methylcellulose, or mixtures thereof. Binders can preferably be present in amounts of 1 to 10 wt%, preferably of 2 to 5 wt%, in particular of 2 to 4 wt% based on the total weight of the dosage form.

Surfactants can preferably be substances which lower the surface tension or the interfacial tension between two phases, thus enabling or supporting the formation of dispersions or working as a solubilizer. Surfactants can preferably be present in amounts of 0.3 to 5 wt%, preferably of 0.5 to 4 wt%, in particular of 0.5 to 3 wt% based on the total weight of the dosage form.

Lubricants are generally used in order to reduce sliding friction. In particular, the intention is to reduce the sliding friction found during tablet pressing between the punch moving up and down in the die and the die wall, on the one hand, and between the edge of the tablet and the die wall, on the other hand. Suitable lubricants are, for example, stearic acid, adipic acid, sodium stearyl fumarate and/or magnesium stearate. Preferably, lubricants can be present in an amount of 0 to up to 5 wt%, more preferably of 0.1 to 4 wt%, in particular of 0.2 to 3 wt% based on the total weight of the dosage form.

Glidants can be used to improve the flowability. Traditionally, talc was used as glidant, but it is nowadays nearly fully replaced by silica, including but not limited to colloidal silica (for example Aerosil^{®}) and fumed silica (for example Cab-o-Sil). Preferably, the glidant can be present in an amount of 0 to up to 3 wt%, more preferably of 0.1 to 2.8 wt%, in particular of 0.2 to 2.5 wt% based on the total weight of the dosage form. Preferably, the silica has a specific surface area of 50 to 400 m²/g, measured by gas adsorption according to Ph. Eur. 6.0, chapter 2.9.26, multipoint method, volumetric determination.

It lies in the nature of pharmaceutical excipients that they sometimes can perform more than one function in a pharmaceutical formulation.

In the context of this invention, in order to provide an unambiguous delimitation, the fiction will therefore preferably apply that a substance which is used as a particular excipient is not simultaneously also used as a further pharmaceutical excipient. For example, microcrystalline cellulose - if used as a filler - is not also used as for example a disintegrant (even though microcrystalline cellulose also exhibits a certain disintegrating effect).

Generally, the term "additional API" refers to any pharmaceutically active substance being described as having an effect in treatment of osteoporosis, preferably to post-menopausal osteoporosis.

In a preferred embodiment, the provision in phase I) of odanacatib and at least one pharmaceutical excipient can be carried out with conventional mixing devices, e.g. in a free fall mixer like Turbula^{®} T 10B (Bachofen AG, Switzerland), Glatt CML 10 cubic mixer. Mixing can be carried out, e.g., for 1 minute to 1 hour, preferably for 5 to 30 minutes, even more preferably 10 to 20 minutes.

In an alternative embodiment, the mixing can be conducted such that either the total amount or a certain part of the odanacatib is mixed with a first part of the at least one pharmaceutical excipient in a mixing device, for example in a high shear or tumbler mixer generating a phase-I-product. After this initial mixing step an additional part of the odanacatib and/ or of the at least one pharmaceutical excipient can be added to phase-I-product, which is followed by an additional mixing step. Optionally any of the addition steps can be performed under constant mixing. This procedure can be repeated until the last part of pharmaceutical excipient(s) is used, preferably one to five times. This kind of mixing can assure an even distribution of active agent resulting in a good CU and provides a phase-I-product for further processing in phase II).

In a further preferred embodiment in phase I) odanacatib is provided with a disintegrant and with a filler. The disintegrant is preferably selected from carboxymethyl cellulose sodium and crosslinked polyvinylpyrrolidone, especially carboxymethyl cellulose sodium. The filler is preferably selected from lactose, microcrystalline cellulose, dextrose and mixtures thereof.

In phase II) granulation of the mixture from phase I) with a granulation liquid comprising a surfactant and optionally at least one further pharmaceutical excipient is carried out. Preferably, an aqueous granulation liquid is used. Regarding to the composition of the granulation liquid it is referred to the explanations given below.

"Granulating" is generally understood to mean the formation of relatively coarse or granular aggregate material as a powder by assembling and/or aggregating finer powder particles (agglomerate formation, or build-up granulation) and/or the formation of finer granules by breaking up coarser aggregates (disintegration, or break-down granulation). The granules can either be generated by wet or dry granulation processes.

In a preferred embodiment an aqueous granulation liquid is used. Generally "aqueous" means that the granulation liquid contains substantial amounts of water. Preferably, the granulation liquid contains solvent, surfactant and optionally a further excipient. The solvent preferably contains water, more preferably from 50 to 100 wt.-% water, in particular 70 to 100 wt.-% water, especially 90 to 100 wt.-% water, based on the total weight of the solvent. In a preferred embodiment the solvent of the granulation liquid consists essentially of water, in particular consists of water.

The surfactant comprised in the granulation liquid can be a substance lowering the surface tension or the interfacial tension between two phases, thus enabling or supporting the formation of dispersions or working as a solubilizer.

Generally, surfactants are composed of a non-polar and a polar part. The non-polar part can be for example an alkyl group, having more than 8 carbon atoms, or an alkyl phenyl group.

The polar part of the surfactant can be composed of various functional groups being suitable to classify the surfactant into the following four categories; i.e.
- non-ionic surfactants having for example one or a plurality of hydroxy or ether group(s) or combinations thereof
- anionic surfactants having a negatively charged polar group such as a carboxylate, a sulfonate, sulfate or phosphate group
- cationic surfactants having a positively charged polar group such as a quaternary ammonium group
- amphoteric or zwitterionic surfactants having both a negatively charged polar group such as a carboxylate and a positively charged polar group such as a quaternary ammonium group.

In a preferred embodiment of the present method the granulation liquid used in phase II) comprises
55 to 99.9 wt% water,
0.1 to 10 wt% surfactant, preferably a surfactant as described herein, in particular SLS,
0 to 10 wt% excipient, preferably a binder, more preferably a binder as described below, and
0 to 44.9 wt% organic solvent, based on the total weight of the granulation liquid.

It is preferred that the amount of organic solvent comprised in the granulation liquid used in phase II) is from 0 to 25 wt%, preferably 0 to 10 wt%, more preferably 0 to 5 wt% based on the total weight of the granulation liquid. It is particularly preferred that the granulation liquid comprises 0% of organic solvent based on the total weight of the granulation liquid.

In a preferred embodiment the surfactant comprised in the granulation liquid from phase II) comprises 8 to 40 carbon atoms, more preferably 10 to 30 carbon atoms, in particular 12 to 22 carbon atoms. It is further preferred that the above carbon atoms are part of the non-polar part of the surfactant.

Examples for surfactants are polyoxyethylene glycol alkyl ethers, polyoxypropylene glycol alkyl ethers, polyoxyethylene glycol sorbitan alkylesters, fatty alcohols such as cetyl and stearyl alcohols, lauryl sulfates such as ammonium or sodium lauryl sulfate, sodium lauryl ether sulfate, linear alkyl benzene sulfonates, alkyltrimethylammonium salts such as cetyl trimethylammonium bromide or cetyl trimethylammonium chloride cetylpyridinium chloride, dimethyldioctadecylammonium bromide, (3-[(3-Cholamidopropyl)dimethylammonio]-1-propanesulfonate), sultaines such as cocamidopropyl hydroxy sultaine, or betaines such as cocamidopropyl betaine or trimethyl glycine betaine.

Further examples of surfactants can be carbomer copolymer, ccacia, cholesterol, glycerol distearate, lecithin, palm oil, poloxamer, sorbitan trioleate, sodium stearate, sodium laurylsulfate, emulsifyfing wax, caprylic acid, glycerol tristearate, trolamine, sucrose stearate, sorbitan monolaurate, sorbitan monooleate, sorbitan monopalmitate, Sorbitan monostearate, sorbitan sequioleate, carbomer interpolymer, coconut oill, cyclodextrine, canola oil, sodium cetostearylsulfate, polyglyceryl 3 diisostearate, polyglyceryl dioleate, polyoxyethylen 50 stearate, polyoxyl 10 oleyl ether, polyoxyl 20 cetostearyl ether, polyoxyl 35 castor oil, polyoxyl 40 hydrogenated castor oil, polyoxyl 40 stearate, polyoxyllaurylether, polyoxylstearylether, polysorbat 20, polysorbat 40, polysorbat 60, polysorbat 80, glyceryl distearate, glyceryl monolinoleate, glyceryl monooleate, glyceryl monostearate, lanolin alcohole, desoxycholic acid, diethanolamine, diethylen glycerolstearate, ethylenglycerolstearate, alpha-Lactalbumin, mono-, di- and triglycerides, palm kernel oil, oleyloleate, oleic acid, monoethanolamine, oleylalcohols, polyoxylstearate, propylenglykol dicaprylatepropylenglycol monocaprylate, propylenglycolmonostearate.

It is further preferred that the surfactant comprised in the granulation liquid from phase II) is an anionic surfactant. Preferably the anion surfactant is selected from alkyl sulfates such as ammonium lauryl sulfate and sodium lauryl sulfate, alkyl-ether sulfates such as sodium laureth sulfate and sodium myreth sulfate, dioctyl sodium sulfosuccinate, perfluorooctanesulfonate, linear alkylbenzene sulfonates such as octylbenzene sulfonates, alkylcarboxylates such as sodium stearate, and alkyl-aryl ether phosphates and alkyl ether phosphate, more preferably from alkyl sulfates such as ammonium lauryl sulfate and sodium lauryl sulfate and linear alkylbenzene sulfonates such as octylbenzene sulfonates, even more preferably from alkyl sulfates such as ammonium lauryl sulfate and sodium lauryl sulfate, especially sodium lauryl sulfate.

In particular, SLS is used as surfactant in the granulation liquid.

In a preferred embodiment the further pharmaceutical excipient used in the granulation liquid of phase II) is selected from fillers, disintegrants, binders, lubricants and glidants. It is particularly preferred that the pharmaceutical excipient is a binder.

The binder preferably comprised in the granulation liquid of phase II) can preferably be a polymer. The polymer that can be comprised in the granulation liquid of phase II) preferably has a glass transition temperature (Tg) of more than 20°C, more preferably 30°C to 150°C, especially 40°C to 150°C. The glass transition temperature is determined in the context of this invention by means of dynamic differential scanning calorimetry (DSC). For this purpose a Mettler Toledo DSC 1 apparatus can be used. The work is performed at a heating rate of 1-20°C/min., preferably 5-15°C/min., and at a cooling rate of 5-25°C, preferably 10-20°C/min.

The granulation liquid of phase II) may, for example, comprise the following hydrophilic polymers as binder: polysaccharides, such as hydroxypropyl methyl cellulose, hydroxypropyl cellulose, hydroxyethyl cellulose, ethyl hydroxyethyl cellulose, methyl cellulose; polyvinylpyrrolidone, polyvinyl acetate, polyvinyl alcohol, polymers of acrylic acid and their salts, polyacrylamide, polymathacrylates, vinyl pyrrolidone/vinyl acetate copolymers, polyalkylene glycols, such as polypropylene glycol or polyethylene glycol, block copolymers of polyethylene glycol, such as block copolymers of polyethylene glycol and polypropylene glycol and mixtures of the polymers mentioned.

More preferred are hydroxypropyl cellulose, hydroxyethyl cellulose, methylcellulose, polyvinyl pyrrolidone, polyvinyl acetate, vinyl pyrrolidone/vinyl acetate copolymers, polymers of acrylic acid and their salts.

Substances particularly preferably used as binder in the granulation fluid of phase II) are polyvinyl pyrrolidone, preferably with a weight average molecular weight of 2,500 to 3,000,000 g/mol, especially 30,000 to 70,000 g/mol, and hydroxypropyl cellulose, preferably with a weight average molecular weight of 30,000 to 1,500,000 g/mol, especially 50,000 to 1,000,000 g/mol, especially hydroxypropyl cellulose (HPC), preferably with a weight average molecular weight of 30,000 to 1,200,000 g/mol, especially 50,000 to 1,000,000 g/mol.

In particular, a granulation liquid comprising SLS, HPC and water is preferred.

The granulation of the mixture of phase I) with the granulation liquid comprising a surfactant and optionally at least one further pharmaceutical excipient can, for example, be performed in a fluidised-bed granulator, such as Glatt® GPCG 3, or a mixer granulator, such as Diosna 1-6.

The granulation takes preferably 1 minute to 1 hour, preferably 2 to 30 minutes.

If wet granulation is performed, an additional drying step can be preferably employed. "Drying" for the purposes of this invention is understood to mean the separation of liquids adhering to solids. Drying generally takes place in conventional drying equipment, such as cabinet or tray dryers, vacuum dryers, fluidised-bed dryers, spray dryers or freeze dryers. The drying and granulation process is preferably performed in one and the same apparatus. The granulates are then dried and optionally screened. In a preferred embodiment drying is conducted at temperatures from 25 to 80°C, preferably from 30 to 70°C, in particular from 35° to 55°C. A suitable granulating machine is, for example, Diosna® P1/6.

In phase III) the granulates from phase II) can preferably be blended with further pharmaceutical excipient(s) and/or further pharmaceutically active agent(s). The further pharmaceutical excipient(s) in phase III) can be preferably selected from the pharmaceutical excipient(s) as described above. More preferably, the further pharmaceutical excipient(s) in phase III) can be selected from filler, disintegrant, lubricant and glidant or a mixture thereof. Preferably the further pharmaceutical excipient(s) in phase III) can be a mixture of filler, disintegrant and lubricant. Alternatively preferred the further pharmaceutical excipient(s) in phase III) can be a lubricant.

The filler used in phase III) can preferably be lactose and/or microcrystalline cellulose, preferably lactose.

The disintegrant used in phase III) can preferably be carboxymethyl cellulose sodium and /or cross-linked polyvinylpyrrolidone.

The lubricant used in phase III) can preferably be magnesium stearate.

In a preferred embodiment the components from phase II) and/or the further pharmaceutical excipient(s) from phase III) can be sieved before being blended. In a preferred embodiment the sieve has a mesh size of 100 to 1000 µm, preferably of 200 to 800 µm.

The blending of the above components can preferably be carried out in a mixer, preferably in a tumble blender.

The blending takes preferably from 1 minute to 1 hour, preferably from 2 to 30 minutes, more preferably from 3 to 10 minutes.

In a preferred embodiment of the invention phase III is optional.

In another preferred embodiment one or more active pharmaceutical ingredients can optionally added during any one of phase I), phase II) and phase III).

The blend from phase III) is processed into a dosage form, preferably an oral dosage form. The oral dosage forms may be single unit dosage from (SUDFs), such as monolithic tablets, troches, lozenges or capsules, or multiple unit dosage forms (MUDFs), such as multi particulate tablets, multi unit pellet system (MUPS), granules/pellets/cores/mini-tablets filled into capsules, sachets, stick packs and other dosage forms for oral administration. The processing of the blend from phase III) can preferably comprise compressing the blend of phase III) into tablets or filling the blend of step c) into capsules or sachets. More preferably the blend of phase III) can be compressed into tablets.

Compressing the blend from phase III) into tablets can be carried out by compressing said mixture on a press, for example on a rotary press, e.g. on a Fette^{®} (Fette GmbH, Germany) or a Riva^{®} Piccola (Riva, Argentina) or on an eccentric press, for example (Korsch EK0). The compression force can preferably range from 1 to 50 kN, preferably 3 to 40 kN.

In another embodiment, the processing of the blend of phase III) to a dosage form can be done by filling the blend of phase III) into capsules, preferably hard gelatine capsules. For this filling of the blend of phase III) into capsules, dependent dosing systems (for example an auger) or preferably independent dosing systems (for example MG2, Matic (IMA)) can be used.

In a preferred embodiment of the invention odanacatib can be present in form of crystalline odanacatib.

The term "crystalline" can be used in the context of this invention to designate the state of solid substances in which the components (atoms, ions or molecules, i.e. in the case of crystalline odanacatib the odanacatib molecules) are arranged in an orderly repeating pattern, extending in all three spatial dimensions and thus exhibit a periodic arrangement over a great range (= long-range order).

In a preferred embodiment odanacatib in the composition may consist of purely crystalline odanacatib. Alternatively, it may also contain small amounts of non-crystalline odanacatib components provided that a defined melting point of crystalline odanacatib can be detected in a DSC. It is preferred that the odanacatib contained in the inventive dosage form can be a mixture containing 85 to 99.999% by weight crystalline odanacatib and 0.001 to 15% by weight non-crystalline odanacatib, more preferably 90 to 99.99% by weight crystalline odanacatib and 0.01 to 10% non-crystalline odanacatib, particularly preferably 95 to 99.9% by weight crystalline odanacatib and 0.1 to 5% non-crystalline odanacatib.

In a preferred embodiment of the invention odanacatib used in phase I) of the method according to the present invention can have an average particle size (D₅₀) of 0.1 to 250 µm, preferably 0.25 to 150 µm, more preferably 0.5 to 75 µm, especially 1.0 to 50 µm. The average particle size can refer to the D₅₀ of the particle size distribution. The average particle size can be determined by means of laser diffractometry. In particular, a Malvern Instruments Mastersizer 2000 can be used to determine the size (preferably wet measurement with ultrasound 60 sec., 2,000 rpm, preferably dispersed in sunflower oil, the evaluation being performed according to Particle RI set to 1.520 and Absorption of 2).

The average particle size (D₅₀), which is also denoted D₅₀-value of the integral volume distribution, is defined in the context of this invention as the particle diameter at which 50% by volume of the particles have a smaller diameter than the diameter which corresponds to the D₅₀-value. Likewise, 50% by volume of the particles have a larger diameter than the D₅₀-value.

Analogously, the D₁₀-value of the integral volume distribution is defined as the particle diameter at which 10% by volume of the particles have a smaller diameter than the diameter which corresponds to the D₁₀-value. In a preferred embodiment the D₁₀-value of the integral volume distribution is from 0.02 to 65 µm, preferably from 0.05 to 40 µm, more preferably from 0.1 to 20 µm and especially from 0.25 to 10 µm.

Analogously, the D₉₀-value of the integral volume distribution is defined as the particle diameter at which 90% by volume of the particles have a smaller diameter than the diameter which corresponds to the D₉₀-value. In a preferred embodiment the D₉₀-value of the integral volume distribution is from 0.3 to 500 µm, preferably from 1 to 300 µm, more preferably from 3 to 200 µm and especially from 5 to 150 µm.

In a preferred embodiment of the invention the present method further comprises the step of
- film-coating the dosage form.

The dosage form) can preferably be a tablet which can be swallowed unchewed (non-film-coated or preferably film-coated).

In a preferred embodiment, the dosage form, preferably a tablet, can be film-coated. For this purpose, in the above step methods known in the art for film-coating tablets may be employed.

Generally, film-coatings can be prepared by using cellulose derivatives, poly(meth)acrylate, polyvinyl pyrrolidone, polyvinyl acetate phthalate, and/or shellac or natural rubbers such as carrageenan.

In a preferred embodiment of the present invention the film-coating can be a film-coating essentially without affecting the release of the active agent.

Preferred examples of film-coatings which do not affect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinyl pyrrolidone (PVP), PVA and mixtures thereof. These polymers can have a weight average molecular weight of 10,000 to 150,000 g/mol.

The film coating can preferably have a thickness of 2 µm to 100 µm, preferably from 20 µm to 60 µm. In case of a coating containing odanacatib, the thickness of the coating is usually 10 µm to 200 µm, preferably from 50 µm to 125 µm.

A further subject of the invention is a dosage from obtainable by the method of the present invention.

Preferably, the dosage form of the invention can be preferably an oral dosage form. It is further preferred that the dosage form of the invention is a capsule or a tablet, in particular a tablet.

It is further preferred that the tablets can have a hardness of 30 to 400 N, more preferred of 50 to 325 N, still more preferred of 75 to 300 N, in particular of 85 to 275 N, wherein the hardness is measured according to Ph. Eur., 6.0, chapter 2.9.8.

In addition, the tablets preferably can have a friability of less than 5%, particularly preferably less than 2%, especially less than 1%. The friability is determined in accordance with Ph. Eur., 7.7, chapter 2.9.7. The friability values generally refer to tablets without coating.

Further, the tablets of the invention preferably have a content uniformity, i.e. a content of active agent(s) which lies within the concentration of 90 to 110%, preferably of 95 to 105%, especially preferred of 98 to 102% of the average content of the active agents(s). The "content uniformity" is determined with a test in accordance with Ph. Eur., 6.0, Chapter 2.9.6. According to that test, the content of the active agents of each individual tablet out of 20 tablets must lie between 90 and 110%, preferably between 95 and 105%, especially between 98 and 102% of the average content of the active agents(s). Therefore, the content of the active drugs in each tablet of the invention differs from the average content of the active agent by at most 10%, preferably by at most 5% and especially by at most 2%.

Hardness, friability and content uniformity are determined from an uncoated tablet. A high drug load composition or high drug load dosage form can be any pharmaceutical composition or dosage form wherein the amount of active pharmaceutical agent is 10wt% or more. In preferred embodiment of the invention the dosage form can preferably comprise 10 to 250 mg odanacatib, more preferably 25 to 100 mg odanacatib, particularly 25 to 75 mg odanacatib, wherein the drug load can preferably be 10 to 30 wt%, more preferably 12 to 25 wt%, even more preferably 12 to 20 wt%, in particular 14 to 18 wt% based on the total weight of the dosage form.

In a preferred embodiment the dosage from of the present invention can preferably comprise the following amounts of components:
- 10 to 30 wt%, preferably 12 to 25 wt%, more preferably 12 to 20 wt%, in particular 14 to 18 wt% odanacatib,
- 50 to 85 wt%, preferably 55 to 80 wt%, in particular 60 to 77 wt% filler,
- 2 to 35 wt%, preferably 3 to 30 wt%, in particular 3 to 25 wt% disintegrant,
- 1 to 10 wt%, preferably 1.5 to 8 wt%, more preferably 2 to 5 wt%, in particular 2 to 4 wt% binder,
- 0.3 to 5 wt%, preferably 0.5 to 4 wt%, in particular 0.5 to 3 wt% surfactant, preferably SLS,
- 0 to 5 wt%, preferably 0.1 to 4 wt%, more preferably 0.2 to 3 wt% lubricant,
- 0 to 3 wt%, preferably 0.1 to 2.8 wt%, more preferably 0.2 to 2.5 wt% glidant,
wherein the wt% are based on the total weight of the dosage form.

In a preferred embodiment the dosage form according to the invention provides an immediate release ("IR") of odanacatib. This means that the release profile of the dosage forms of the invention according to USP app. II (paddle, 900 ml, 0.1 n HCl, pH 1.2, 0.5% SLS, 50 rpm, 37°C) after 15 minutes preferably indicates a content release of 50 to 80%, preferably 55 to 65%.

A further subject of the invention is the use of a granulation liquid containing a surfactant for the preparation of an oral dosage form for immediate release comprising a cathepsin-inhibitor.

In a preferred embodiment a granulation liquid comprising 55 to 99.9 wt% water, and 0.1 to 10 wt% surfactant, and 0 to 10 wt% excipient and 0 to 44.9 wt% organic solvent based on the total weight of the granulation liquid can be used for the preparation of an oral dosage form for immediate release comprising a cathepsin-inhibitor. It is further preferred that the amount of organic solvent comprised in the granulation liquid is from 0 to 25 wt%, preferably 0 to 10 wt%, more preferably 0 to 5 wt% based on the total weight of the granulation liquid. It is particularly preferred that the granulation liquid does not comprise any organic solvent.

The invention will now be explained with reference to the following examples.

### Example 1

Odanacatib, a blend (1:1) of microcrystalline cellulose (mcc) and lactose (Granulac), half of the cross-linked carboxymethyl cellulose sodium (Primellose) were mixed together for 15 minutes at 23 rpm in a Turbular TB10. To a 3% aqueous solution of hydroxypropyl cellulose (HPC EF) Sodiumlauryl sulfate (SLS) was added. Granulates were prepared by adding the granulation liquid to the mixture containing odanacatib while stirring, drying at 40°C in the dry oven for 2 hours and sieving over a mesh size 800 µm sieve. Magnesium stearate, the second half of cross-linked carboxymethyl cellulose sodium (Primellose) and the residual lactose (Granulac) was added to granulates and the mixture was blended for 3 minutes at 23 rpm. The final blend was compressed on an eccentric press (Korsch EK0) wherein the tablets, having a drug load of 14.29%, each contained

| **API + Excipient** | **[mg / DF]** | **[% / DF]** | |
|---|---|---|---|
| Odanacatib | 50.00 | 14.29 | Phase I |
| Granulac | 95.00 | 27.14 | |
| mcc, 102 | 95.00 | 27.14 | |
| Primellose | 7.00 | 2.00 | |
| HPC EF | 11.00 | 3.14 | Phase II |
| SLS | 7.50 | 2.14 | |
| Water q.s. | | - | |
| Primellose | 7.00 | 2.00 | Phase III |
| Mg stearat | 2.00 | 0.57 | |
| Granulac | 75.50 | 21.57 | |
| **Total** | **350** | | |

### Example 2

Odanacatib, dextrose (Emdex), half of the cross-linked carboxymethyl cellulose sodium (Primellose) were mixed together for 15 minutes at 23 rpm in a Turbular TB10. To a 3% aqueous solution of hydroxypropyl cellulose (HPC EF) Sodiumlauryl sulphate (SLS) was added. Granulates were prepared by adding the granulation liquid to the mixture containing odanacatib while stirring, drying at 40°C in the dry oven for 2 hours and sieving over a mesh size 800 µm sieve. Magnesium stearate, the second half of cross-linked carboxymethyl cellulose sodium (Primellose) and lactose (Granulac) was added to granulates and the mixture was blended for 3 minutes at 23 rpm. The final blend was compressed on an eccentric press (Korsch EK0) wherein the tablets, having a drug load of 14.29%, each contained

| **API + Excipient** | **[mg / DF]** | **[% / DF]** | |
|---|---|---|---|
| Odanacatib | 50.00 | 14.29 | Phase I |
| Emdex | 192.00 | 54.86 | |
| Primellose | 7.00 | 2.00 | |
| HPC EF | 9.00 | 2.57 | Phase II |
| SLS | 7.50 | 2.14 | |
| Water q.s. | | - | |
| Primellose | 7.00 | 2.00 | Phase III |
| Mg stearate | 2.00 | 0.57 | |
| Granulac | 75.50 | 21.57 | |
| **Total** | **350** | | |

### Example 3

Odanacatib, a part of cross-linked polyvinylpyrrolidone (CL, 63 mg), a part of lactose (Granulac, 70 mg) were mixed together for 15 minutes at 23 rpm in a Turbular TB10. To a 3% aqueous solution of polyvinylpyrrolidone (PVP) Sodiumlauryl sulfate (SLS) was added. Granulates were prepared by adding the granulation liquid to the mixture containing odanacatib while stirring, drying at 40°C in the dry oven for 2 hours and sieving over a mesh size 800 µm sieve. Magnesium stearate, residual lactose (Granulac), microcrystalline cellulose/highly dispersed silicon dioxide (Prosolv) and residual cross-linked polyvinylpyrrolidone (CL) was added to granulates and the mixture was blended for 3 minutes at 23 rpm. The final blend was compressed on an eccentric press (Korsch EK0) wherein the tablets, having a drug load of 14.29%, each contained

| **API + Excipient** | **[mg / DF]** | **[% / DF]** | |
|---|---|---|---|
| Oda | 50.00 | 14.29 | Phase I |
| Granulac | 70.00 | 20.00 | |
| CL | 63.00 | 18.00 | |
| PVP 30 | 13.00 | 3.71 | Phase II |
| SLS | 2.00 | 0.57 | |
| Water q.s. | | - | |
| CL | 20.00 | 5.71 | Phase III |
| Prosolv (98% microcrystalline cellulose and 2 % highly dispersed silicon dioxide) | 66.00 | 18.86 | |
| Granulac | 66.00 | 18.86 | |
| Mg stearat q.s. | | - | |
| **Total** | **350** | | |

### Example 4, "Reference Example"

### (Example 2 from WO 2009/140105)

The reference formulation comprised 44.44% of a spray-dried product consisting of 15% odanacatib and 85% of HPMCAS-HF, 45.31% Lactose (Flowlac), 6% croscarmellose sodium, 2% sodiumlauryl sulphate (SLS), 1% Carb-o-Sil, 1.25% Mg-stearate and was produced by blending the spray-dried product with lactose, croscarmellose sodium, SLS, Carb-o-Sil and half of the magnesium stearate. The resulting product was compacted. The resulting granulated composition was milled through a 1 mm screen, and the milled granulated material was blended for 5 minutes with the remaining half of magnesium stearate, finally tablets were compressed. The tablets, having a drug load of 6.667%, each contained

| **API + Excipient** | [%] |
|---|---|
| 15% Odanacatib + 85% HPMCAS-HF | 44.44 |
| Lactose (Flowlac) | 45.31 |
| Croscarmellose sodium | 6.00 |
| Sodiumlauryl sulfate (SLS) | 2.00 |
| Silica (Carb-o-Sil) | 1.00 |
| Mg stearat | 1.25 |
| **Total** | **100** |

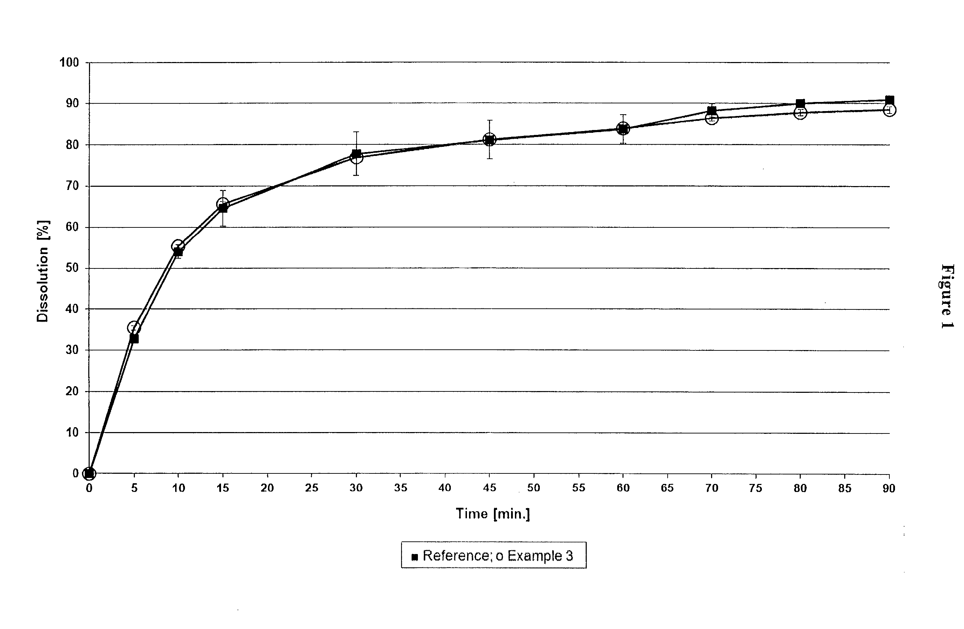

Figure 1 shows the dissolution of present Example 3 in comparison with Example 4 ("Reference") measured with conditions of 900 mL 0.1N HCl pH 1.2 with 0.5% SDS - 37°C - 50 rpm paddle (USP app. II). It can be seen that the dissolution profile of the formulation disclosed in Example 3, having a significantly higher drug load than the formulation in Example 4, is similar to the dissolution profile of Example 4.

## Claims

1. Method for producing a dosage form comprising odanacatib comprising the phases of
phase I) providing odanacatib and at least one pharmaceutical excipient; and
phase II) granulating the mixture of phase I) with a granulation liquid comprising a surfactant and optionally at least one further pharmaceutical excipient; and
phase III) blending the granulates of phase II) with further pharmaceutical excipient(s); and
finally processing the blend of phase III) into a dosage form.

2. Method according to claim 1, wherein odanacatib is present in crystalline form.

3. Method according to claim 1 or 2, wherein the average particle size (D₅₀) of the odanacatib used in phase I) is 1.0 to 50 µm.

4. Method according to any one of claims 1 to 3, wherein the granulation liquid used in phase II) comprises
55 to 99.9 wt% water,
0.1 to 10 wt% surfactant
0 to 10 wt% excipient and
0 to 44.9 wt% organic solvent,
based on the total weight of the granulation liquid.

5. Method according to any one of claims 1 to 4, wherein the surfactant is an anionic surfactant, preferably Sodiumlauryl sulfate.

6. Method according to any one of claims 1 to 5, wherein the at least one further pharmaceutical excipient in phase II) is selected from fillers, disintegrants, binders, surfactants, lubricants and glidants.

7. Method according to any one of claims 1 to 6, wherein the method further comprises the step of film-coating the dosage.

8. Granulates obtainable by phases I) and II) of the method according to claims 1 to 7.

9. Dosage form obtainable by a method according to any one of claims 1 to 8.

10. Dosage form according to claim 9, wherein the dosage form is a tablet, preferably a tablet having a content uniformity of 95 to 105%, a friability of less than 5% and/or a hardness of 50 to 325 N.

11. Dosage form according to claim 9 or 10, wherein the dosage from comprises 25 to 100 mg odanacatib and wherein the drug load is 10 to 30%.

12. Dosage form according to any one of claims 9 to 11, wherein the dosage form comprises
10 to 30 wt% odanacatib,
50 to 85 wt% filler,
3 to 25 wt% disintegrant
2 to 5 wt% binder
0.3 to 5 wt% surfactant
0 to 5 wt% lubricant
0 to 3 wt. % glidant,
based on the total weight of the dosage form.

13. Dosage form according to any one of claims 9 to 12, wherein the dissolution of odanacatib is 50 to 80% after 15 minutes.

14. Use of an aqueous granulation liquid containing a surfactant for the preparation of an oral dosage form for immediate release comprising a cathepsin-inhibitor.

15. Use according to claim 14, wherein the granulation liquid comprises
55 to 99.9 wt% water,
0.1 to 10 wt% surfactant
0 to 10 wt% excipient and
0 to 44.9 wt% organic solvent,
based on the total weight of the granulation liquid.
